# EUROPEAN PATENT APPLICATION

(11) **EP 4 821 501 A1**
(43) Date of publication of application: **14.10.2026**
(21) Application number: 24901153.7
(22) Date of filing: 09.12.2024
(51) Int. Cl.: A61K 36/535, A61P 11/00

(54) **COMPOSITION FOR TREATING RESPIRATORY DISEASES COMPRISING PERILLA LEAF EXTRACT**

(30) Priority: 08.12.2023 KR 20230177876
(71) Applicant: CorestemChemon Inc., Seongnam-si, Gyeonggi-do 13486 (KR)
(72) Inventor: KWON, Oh Young, Siheung-si Gyeonggi-do 14967 (KR); SONG, Si Whan, Yongin-si Gyeonggi-do 16975 (KR)
(74) Representative: Cabinet Becker et Associés
(86) International application number: PCT/KR2024/020023
(87) International publication number: WO 2025/121967

(57) **Abstract**

The present invention relates to a pharmaceutical composition for treating and preventing allergic bronchitis, comprising a perilla leaf extract as an active ingredient, and a preparation method therefor, and may be used as functional health food and feed compositions for preventing and alleviating allergic bronchitis.

## Description

### [Technical Field]

The present disclosure relates to a composition that exhibits a therapeutic activity for respiratory diseases, and more particularly, to a pharmaceutical composition and a health functional food composition having an effect for treating or preventing respiratory diseases, including a perilla leaf extract as an active ingredient.

### [Background Art]

Perilla is emerging as a high-income crop, with rising demand comparable to sesame oil while receiving attention for previously unknown effects, such as prevention of anemia, vascular health, alleviated cholesterol levels, and immunity boosting. The perilla has an advantage of enabling eco-friendly cultivation without the use of pesticides due to low incidence of pests and diseases, as well as little damage from wild animals, and may be easily cultivated at a relatively low cost in aging rural areas and thus contributes to generating farm income.

Domestic production of perilla oil continues to expand as exports of the perilla oil are currently diversifying to the United States, Singapore, and Taiwan following Japan, and perilla oil contains at least 60% of omega-3-based alpha-linolenic acid, and is emerging as a food ingredient that may be exported globally as a food with health functionality in addition to the unique flavor of vegetable oil. In 2021, perilla oil was approved as a functional labeling food in Japan, and was found that taking 4.7 g a day is good for hypertension.

A mixed extract of perilla and purslane (No. 2021-11) has been approved for its functionality capable of helping to improve an immune function. Although the perilla leaves have been proven to be useful in traditional Korean medicine over a long period of time, the perilla leaves themselves have not been utilized in various compositions for a therapeutic activity of respiratory diseases.

Accordingly, the present inventors conducted experiments on an effect of inhibiting respiratory diseases using a perilla leaf extract as an active ingredient and verified the effect with data. The present inventors intend to provide an invention relating to a pharmaceutical composition for treating or preventing respiratory diseases including a perilla leaf extract as an active ingredient.

### [Disclosure]

### [Technical Problem]

A main object of the present disclosure is to provide a pharmaceutical composition and a health functional food composition containing a perilla leaf extract as an active ingredient that exhibits an effect of treating or preventing respiratory diseases.

### [Technical Solution]

The terms used in the present disclosure are used for the purpose of description only, and should not be construed to be limited. A singular expression includes a plural expression unless the context clearly indicates otherwise. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which exemplary embodiments pertain. Terms which are defined in a generally used dictionary should be interpreted to have the same meaning as the meaning in the context of the related art, and are not interpreted as an ideal meaning or excessively formal meanings unless clearly defined in the present application.

The present disclosure provides a pharmaceutical composition for treating or preventing respiratory diseases including a perilla leaf extract as an active ingredient.

Preferably, the extract may be an ethanol extract, but is not limited thereto.

The pharmaceutical composition according to the present disclosure may further include suitable carriers, excipients, and diluents, which are commonly used in the preparation of the pharmaceutical composition. The excipients may be, for example, one or more selected from the group consisting of diluents, binders, disintegrants, lubricants, adsorbents, moisturizers, film-coating materials, and controlled-release additives.

The pharmaceutical composition according to the present disclosure may be formulated and used in the form of external preparations, such as powders, granules, sustained-release granules, enteric-coated granules, liquids, eye drops, elixirs, emulsions, suspensions, alcohols, troches, aromatic water, limonades, tablets, sustained-release tablets, enteric-coated tablets, sublingual tablets, hard capsules, soft capsules, sustained-release capsules, enteric-coated capsules, pills, tinctures, soft extracts, dry extracts, fluid extracts, injections, capsules, perfusates, sticky ointments, lotions, pastes, sprays, inhalants, patches, sterile injectable solutions, or aerosols, according to conventional methods, respectively. The external preparations may have formulations such as creams, gels, patches, sprays, ointments, sticky ointments, lotions, liniments, pastes, or cataplasmas.

The carriers, the excipients, and the diluents that may be included in the pharmaceutical composition according to the present disclosure may include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

When the pharmaceutical composition is formulated, the formulations may be prepared by using diluents or excipients, such as a filler, an extender, a binder, a wetting agent, a disintegrating agent, and a surfactant, which are generally used.

The additives of the tablets, powders, granules, capsules, pills and troches according to the present disclosure may be used with excipients such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium bicarbonate, refined lanolin, microcrystalline cellulose, dextrin, sodium alginate, methylcellulose, sodium carboxymethyl cellulose, kaolin, urea, colloidal silica gel, hydroxypropyl starch, hydroxypropyl methyl cellulose (HPMC) 1928, HPMC 2208, HPMC 2906, HPMC 2910, propylene glycol, casein, calcium lactate and Primojel; and binders such as gelatin, arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, refined shellac, starch, hydroxypropyl cellulose, hydroxypropyl methylcellulose, polyvinyl alcohol, and polyvinyl pyrrolidone. In addition, the additives may be used with disintegrants such as hydroxypropyl methylcellulose, corn starch, agar powder, methylcellulose, bentonite, hydroxypropyl starch, sodium carboxymethylcellulose, sodium alginate, carboxymethyl cellulose calcium, calcium citrate, sodium lauryl sulfate, anhydrous silicic acid, 1-hydroxypropyl cellulose, dextran, ion exchange resin, polyvinyl acetate, formaldehyde-treated casein and gelatin, alginic acid, amylose, guar gum, baking soda, polyvinyl pyrrolidone, calcium phosphate, gelled starch, arabic gum, amylopectin, pectin, sodium polyphosphate, ethyl cellulose, white sugar, magnesium aluminum silicate, di-sorbitol solution, and light anhydrous silicic acid; and lubricants, such as calcium stearate, magnesium stearate, stearic acid, hydrogenated vegetable oil, talc, lycopodium, kaolin, petrolatum, sodium stearate, cocoa butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, macrogol, synthetic aluminum silicate, anhydrous silicic acid, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

Additives in the liquids according to the present disclosure may be used with water, diluted hydrochloric acid, diluted sulfuric acid, sodium citrate, monostearate sucroses, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamin, polyvinylpyrrolidone, ethylcellulose, sodium carboxymethylcellulose, etc.

The syrups according to the present disclosure may be used with a solution of white sugar, other sugars or sweeteners, etc., and may also be used with a flavoring agent, a coloring agent, a preservative, a stabilizer, a suspending agent, an emulsifier, a thickener, etc., as needed.

In the emulsions according to the present disclosure, purified water may be used, and an emulsifier, a preservative, a stabilizer, a fragrance, and the like may be used as needed.

The suspensions according to the present disclosure may be used with suspending agents, such as acacia, tragacanth, methylcellulose, carboxymethylcellulose, sodium carboxymethylcellulose, microcrystalline cellulose, sodium alginate, hydroxypropylmethylcellulose (HPMC), HPMC 1828, HPMC 2906, HPMC 2910, and the like, and may be used with surfactants, preservatives, stabilizers, colorants, and fragrances as needed.

The injections according to the present disclosure may include: solvents such as distilled water for injection, 0.9% sodium chloride injection, Ringer's injection, dextrose injection, dextrose + sodium chloride injection, PEG, lactated Ringer's injection, ethanol, propylene glycol, nonvolatile oils such as sesame oil, cottonseed oil, peanut oil, soybean oil, and corn oil, ethyl oleate, isopropyl myristate, and benzene benzoate; solubilizers such as sodium benzoate, sodium salicylate, sodium acetate, urea, urethane, monoethylacetamide, butazolidine, propylene glycol, Tweens, nicotinamide, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃), carbon dioxide, sodium metabisulfite (Na₂S₂O₅), sodium sulfite (Na₂SO₃), nitrogen gas (N₂), and ethylenediaminetetraacetic acid; sulfating agents such as sodium bisulfide 0.1%, sodium formaldehyde sulfoxylate, thiourea, disodium ethylenediaminetetraacetate, and acetone sodium bisulfite; analgesics such as benzyl alcohol, chlorobutanol, procaine hydrochloride, glucose, and calcium gluconate; and suspending agents such as CMC sodium, sodium alginate, Tween 80, and aluminum monostearate.

The suppositories according to the present disclosure may be used with base materials, such as cocoa butter, lanolin, witepsol, polyethylene glycol, glycerogelatin, methylcellulose, carboxymethylcellulose, a mixture of stearic acid and oleic acid, subanal, cottonseed oil, peanut oil, palm oil, cocoa butter + cholesterol, lecithin, lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydroxocote SP, S-70-XXA, S-70-XX75 (S-70-XX95), Hydrokote 25, Hydrokote 711, Idropostal, Massa estrarium (A, AS, B, C, D, E, I, T), Massa-MF, Masupol, Masupol-15, Neosupostal-N, Paramound-B, Suppocire (OSI, OSIX, A, B, C, D, H, L), suppository base type IV (AB, B, A, BC, BBG, E, BGF, C, D, 299), Supostal (N, Es), Wecovi (W, R, S, M, Fs), and Tezester triglyceride bases (TG-95, MA, 57).

Solid formulations for oral administration include tablets, pills, powders, granules, capsules, etc., and these solid formulations are prepared by mixing the extract with at least one excipient, such as starch, calcium carbonate, sucrose or lactose, gelatin, etc. Further, lubricants such as magnesium stearate and talc may be used in addition to simple excipients.

Liquid formulations for oral administration may correspond to suspensions, oral liquids, emulsions, syrups, and the like, and may include various excipients, such as wetting agents, sweeteners, flavoring agents, preservatives, and the like, in addition to water and liquid paraffin which are commonly used as simple diluents. Formulations for parenteral administration include sterile aqueous solutions, non-aqueous solvents, suspensions, emulsions, lyophilized preparations, and suppositories. As the non-aqueous solution and the suspension, propylene glycol, polyethylene glycol, vegetable oil such as olive oil, injectable ester such as ethyl oleate, and the like may be used.

The pharmaceutical composition of the present disclosure is administered in a pharmaceutically effective amount. As used in the present disclosure, the "pharmaceutically effective amount" refers to an amount sufficient to treat a disease at a reasonable benefit/risk ratio applicable to medical treatment. The effective amount level may be determined according to factors including the type and severity of a patient's disease, the activity of a drug, the sensitivity to a drug, a time of administration, a route of administration, an excretion rate, duration of treatment, and simultaneously used drugs, and other factors well-known in the medical field.

The pharmaceutical composition according to the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art to which the present disclosure pertains.

The pharmaceutical composition of the present disclosure may be administered to a subject through various routes. All methods of administration may be expected, and for example, the pharmaceutical composition may be administered by oral administration, subcutaneous injection, intraperitoneal administration, intravenous injection, intramuscular injection, intrathecal injection, sublingual administration, buccal administration, rectal insertion, vaginal insertion, ocular administration, otic administration, nasal administration, inhalation, spraying through the mouth or nose, dermal administration, transdermal administration, etc.

The pharmaceutical composition of the present disclosure is determined according to a type of drug as an active ingredient in addition to many related factors, such as a disease to be treated, an administration route, the age, gender, and body weight of a patient, the severity of the disease, etc.

As used in the present disclosure, the "subject" refers to a subject in need of treatment for diseases, and more particularly, refers to mammals such as humans or non-human primates, mice, rats, dogs, cats, horse, and cow.

As used in the present disclosure, the "administration" means providing a predetermined composition of the present disclosure to a subject by any suitable method. As used in the present disclosure, the "prevention" means any action of suppressing or delaying the onset of a target disease, the "treatment" means any action of improving or beneficially changing a target disease and its resulting metabolic disorder symptoms by administering the pharmaceutical composition according to the present disclosure, and the "alleviation" means any action of reducing parameters related to a target disease, for example, the degree of symptoms, by administering the composition according to the present disclosure.

As used in the present disclosure, the perilla leaf extract may exhibit an effect through a mechanism that inhibits lymphocyte production or the expression of immunoglobulin, and the immunoglobulin may be IgE, but is not limited thereto.

The present disclosure also provides a health functional food composition for preventing or alleviating respiratory diseases including the perilla leaf extract.

As used in the present disclosure, the "food" means natural products or processed products containing one or more nutrients, and preferably, means a condition that may be eaten directly through a certain amount of processing process, and as a general meaning, means including all of health functional foods, beverages, food additives, beverage additives, etc.

As used in the present disclosure, the "functional food" is the same term as "a food for special health use (FoSHU)" and refers to a food with high medical or health effects that is processed to efficiently exhibit bio-regulatory functions in addition to nutritional supply, and may be prepared in the form of tablets, capsules, pills, granules, powders, liquids, flakes, pastes, syrups, gels, jellies, bars, or film formulations. Here, the 'functionality' means regulating nutrients for the structure and function of the human body or obtaining useful effects for health applications such as physiological actions, etc.

When the perilla leaf extract of the present disclosure is used as the food additive, the perilla leaf extract may be added as it is or used with other foods or food ingredients, and may be appropriately used according to a general method. The mixed amount of the active ingredients may be suitably determined according to a purpose of use (preventive, healthy, or therapeutic treatment). In general, in the preparation of foods or beverages, the perilla leaf extract of the present disclosure may be added in an amount of 15 wt% or less, or 10 wt% or less with respect to raw materials. However, in the case of long-term intake for the purpose of health and hygiene or for the purpose of health control, the amount may be equal to or greater than the range, and has no problem in terms of safety, so that the active ingredients may be used even in an amount above the range.

The kind of food is not particularly limited. Examples of the food which may be added with the materials include meat, sausages, bread, chocolate, candies, snacks, confectionery, pizza, ramen, other noodles, gums, dairy products including ice cream, various soups, beverages, tea, drinks, alcohol drinks, vitamin complex, and the like, and include all health functional foods in an accepted meaning.

In the health beverage composition according to the present disclosure, like general beverages, various flavoring agents, natural carbohydrates, or the like may be added as an additional ingredient. The above-mentioned natural carbohydrates include monosaccharides such as glucose and fructose, disaccharides such as maltose and sucrose, polysaccharides such as dextrin and cyclodextrin, and sugar alcohols such as xylitol, sorbitol and erythritol. As the sweetening agent, natural sweetening agents such as thaumatin and stevia extracts, synthetic sweetening agents such as saccharin and aspartame, and the like may be used. A ratio of the natural carbohydrate may be generally about 0.01 to 0.20 g or about 0.04 to 0.10 g per 100 mL of the composition of the present disclosure.

In addition to the ingredients, the composition of the present disclosure may contain various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, or the like. In addition, the composition of the present disclosure may include pulps for preparing natural fruit juices, fruit juice beverages or vegetable beverages. These ingredients may be used independently or in combination. Although the ratio of the additives is not very important, generally, the ratio is selected in the range of 0.01 to 0.20 part by weight per 100 parts by weight of the composition of the present disclosure.

The present disclosure also provides a feed composition for treating and alleviating respiratory diseases including the perilla leaf extract.

As used in the present disclosure, the term 'feed' means any natural or artificial diet, one-meal diet, or ingredients of the one-meal diet to be eaten, ingested, and digested by livestock or suitable thereto.

The type of feed is not particularly limited, and may use feeds commonly used in the art. Non-limiting examples of the feed may include vegetable feeds, such as grains, root fruits, food processing by-products, algae, fibers, pharmaceutical by-products, oils and fats, starches, cucurbitaceous plants or grain by-products; and animal feeds such as proteins, inorganic materials, oils and fats, minerals, oils and fats, single-cell proteins, animal planktons, foods, etc. These feeds may be used alone or in combination of two or more kinds.

### [Advantageous Effects]

According to the present disclosure, the perilla leaf extract exhibits a therapeutic effect for respiratory diseases, and thus can be used as a pharmaceutical composition and a health functional food composition for treating or preventing respiratory diseases containing the perilla leaf extract as an active ingredient.

### [Description of Drawings]

FIG. 1 shows changes in the total number of leukocytes, lymphocytes, and eosinophils in experimental animals according to administration of a perilla leaf extract.
FIG. 2 shows changes in serum IgE of experimental animals according to administration of a perilla leaf extract.

### [Best Mode]

Hereinafter, preferred Examples will be proposed in order to help in understanding of the present disclosure. However, the following Examples are only provided to more easily understand the present disclosure, and the contents of the present disclosure are not limited by the following Examples.

### Preparation of perilla leaf extract

A raw material was washed and then perilla leaves were blanched in 100°C hot water for 1 to 2 minutes and dried in low-temperature hot air at 45°C for 5 days. Thereafter, the leaves were selected, the stem portions were removed, and then ground, and 100 g of dried perilla leaves were extracted by stirring at 100 rpm at room temperature for 24 hours using 1,900 g of 70% ethanol as a solvent. The extract was filtered through a 5 to 8 µm filter, concentrated to about 80%, and centrifuged at 11,000 rpm for 30 minutes, and then a supernatant was discarded, and the extract was suspended in 400 g of distilled water and vortexed. Thereafter, the extract was sonicated at 65°C for 30 minutes, filtered through a 5 to 8 µm filter, dried with hot air at 82°C for 3 days, and then finely ground.

### Preparation of respiratory disease-induced animal models

The experiment was approved by the Experimental Animal Operations Committee of the Non-Clinical Research Institute of CORESTEMCHEMON Inc. in accordance with the Animal Protection Act and the Laboratory Animal Act (Review No.: 2023-0535).

10-week-old female Specific Pathogen-Free (SPF) BALB/c mice (BALB/cAnNCrljOri) (Orient Bio, Daejeon, Korea) were received and freely ingested rodent solid feed (Teklad certified irradiated global 18% protein rodent diet, 2918C, Inotiv, USA) and tap water disinfected with a UV sterilizer and a microfiltration device in a polycarbonate breeding cage. The mice were acclimatized for about 7 days in a laboratory environment at a temperature of 22 ± 3°C, humidity of 55 ± 15%, and a 12-hour night-day cycle (lighting time: 8:00 AM), their general health conditions were observed, and then healthy subjects were selected. Subsequently, the selected experimental animals were divided into four groups of G1, G2, G3, and G4.

G1 and G2 were divided by measuring and ranking the body weights of animals determined to be healthy within the experimental group, and randomly distributed as in the experimental group configuration so that average body weights for each group were uniformly distributed. G3 and G4 were divided by measuring and ranking the body weights of animals determined to be healthy among the remaining sensitized animals (ovalbumin 10 µg/0.1 mL/head, total 2 times), and randomly distributed as in the experimental group configuration so that the average body weights of each group were uniformly distributed.

10 mL/kg of the perilla leaf extract and 20 mL/kg of PBS, which was an excipient as a control group, were administered to the experimental animals at a frequency of once a day for 30 days, respectively, and then administered directly into the stomach using a syringe tube equipped with a zonde after correcting the scapular regions of the experimental animals with a hand. The dose and conditions for each experimental group were listed in Table 1.

One hour after administration of the perilla leaf extract, one or two animals in each experimental group were placed in the same chamber, the lid was closed, and then 10 mL (10 mg/mL) of 1% ovalbumin was sprayed into the chamber through a silicone hose connected to a nebulizer to induce allergic bronchitis. The ovalbumin was sprayed three times a week for 60 minutes for four weeks, and the ovalbumin was sprayed for 4 weeks while the perilla leaf extract and the PBS were administered.

**[Table 1]**

| Group | Number of subjects | Dose (mg/kg) | Dosing volume (mg/kg) | Administered substance |
|---|---|---|---|---|
| G1 | 5 | - | 20 | PBS |
| G2 | 5 | - | 20 | |
| G3 | 3 | 300 | 10 | Perilla leaf extract |
| G4 | 3 | 1000 | 10 | |

### Analysis of immune cell count in bronchoalveolar lavage fluid

To collect bronchoalveolar lavage fluids (BALF) from all experiment groups, the experimental animals were euthanized and then their thoracic cavities were opened to expose their lungs. Cold PBS was prepared in a 1 mL syringe connected to a 3-way cock, a needle was inserted into the upper airway, and then the airway and the needle were tied with a string to be fixed. 700 µL of the cold PBS was injected with a syringe, 500 µL of the lavage fluid was recovered through an opposite valve and transferred to a tube, and then 500 µL of the cold PBS was added and 300 µL of the lavage fluid was repetitively recovered twice to obtain approximately 1mL of a bronchoalveolar lavage fluid. The obtained lavage fluid was centrifuged at 1,000 rpm for 10 minutes at 4°C to separate a pellet and a supernatant, and the pellet was resuspended in 500 µL of PBS, and then the immune cell count was analyzed using ADVIA 2120i (SEMENS, USA).

### Serum separation

The experimental animals in all the experimental groups were laparotomized under respiratory anesthesia and then the blood was collected from the saphenous vein. The collected blood was placed in an SST tube and left at room temperature for 15 to 20 minutes to be coagulated, and then the serum was separated by centrifuging at 3,000 rpm for 10 minutes. The separated serum was placed in a tube and stored at -70°C until use for analysis.

### Statistical analysis

Statistical analysis was performed using SPSS statistics 12.0K for medical science. The comparison between a normal group (G1) and a negative control group (G2) in parametric multiple comparison procedures was performed to determine the induction of allergic bronchitis, and the analysis method was performed by a Student's t-test. ONE-WAY ANOVA was performed to compare the negative control group (G2) and perilla leaf extract administration groups (G3-G4), and as a post-hoc test, a Duncan's test was performed for homogeneity of variance and a Dunnett's test was performed for non-homogeneity of variance.

### Example 1. Changes in leukocytes, lymphocytes, and eosinophils in animal model according to treatment with perilla leaf extract

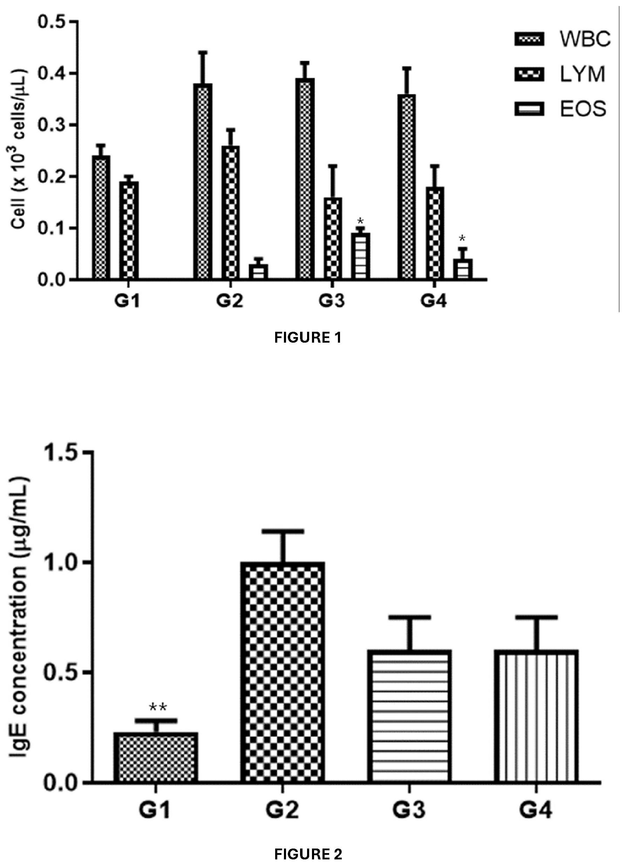

On the last day of administration, experimental animals were sacrificed to obtain a bronchoalveolar lavage fluid, and then leukocytes (WBC), lymphocytes (LYM), and eosinophils (EOS) were measured using a hematological test measurement device (FIG. 1).

As the BALF hematological analysis result, when being compared to the normal group (G1), in the negative control group (G2), significant increases of 56.20%, 31.96%, and 1300.00% were statistically measured for white blood cells (WBC), lymphocytes (LYM), and eosinophils (EOS), respectively, but the EOS were not significant compared to the normal group (G1). In the white blood cells (WBC), no significant change was observed in the perilla leaf extract administration groups (G3-G4) compared to the negative control group (G2). In the lymphocytes (LYM), in the perilla leaf extract administration groups (G3-G4), a decreasing trend of 38.80% and 29.69% was observed, respectively, which was not significant compared to the negative control group (G2). In the eosinophils (EOS), a significant increase was observed in the perilla leaf extract administration groups (G3-G4) compared to the negative control group (G2) (p < 0.05).

### Example 2. Changes in serum IgE in animal model according to administration of perilla leaf extract

On the last day of administration, the blood was collected from all administration groups, and then serum was separated from the blood, and an IgE assay (Invitrogen, Cat No. EMIGHE) was performed (FIG. 2).

As a result of measuring serum IgE, a statistically significant increase was observed in the negative control group (G2) compared to the normal group (G1) (P < 0.01). In the perilla leaf extract administration groups (G3-G4), a decrease in IgE of 38.27% and 33.82% was observed, respectively, which was not statistically significant compared to the negative control group (G2).

## Claims

1. A pharmaceutical composition for treating or preventing allergic bronchitis comprising a perilla leaf extract as an active ingredient.

2. The pharmaceutical composition of claim 1, wherein the perilla leaf extract is extracted with 70% ethanol.

3. The pharmaceutical composition of claim 1, wherein the perilla leaf extract is extracted by including a blanching process before extraction.

4. The pharmaceutical composition of claim 1, wherein the perilla leaf extract exhibits effects through a mechanism of inhibiting lymphocyte production or immunoglobulin expression.

5. The pharmaceutical composition of claim 4, wherein the immunoglobulin is IgE.

6. A method for treating allergic bronchitis comprising administering a pharmaceutical composition of any one of claims 1 to 5 to a subject.

7. A method for inhibiting lymphocyte production or immunoglobulin expression comprising administering a pharmaceutical composition of any one of claims 1 to 5 to a subject.

8. The method of claim 7, wherein the immunoglobulin is IgE.

9. A health functional food composition for preventing or alleviating allergic bronchitis comprising a perilla leaf extract.

10. A feed composition for preventing or alleviating allergic bronchitis comprising a perilla leaf extract.
